# EUROPEAN PATENT SPECIFICATION

(11) **EP 1 389 085 B1**
(45) Date of publication and mention of the grant of the patent: **18.04.2007**
(21) Application number: 02723235.4
(22) Date of filing: 25.02.2002
(51) Int. Cl.: A61Q 1/04, A61Q 1/06, A61K 8/18

(54) **METHOD FOR IMPROVING THE PROPERTIES OF TRANSFER RESISTANT LIP COMPOSITIONS**
VERFAHREN ZUR VERBESSERUNG DER EIGENSCHAFTEN VON ABDRUCKFESTEN LIPPENPRÄPARATEN
PROCEDE POUR AMELIORE LES PROPRIETES DES ROUGES A LEVRES RESISTANT A L'ABRASION

(30) Priority: 27.02.2001 US 271849 P
(43) Date of publication of application: 18.02.2004
(73) Proprietor: Revlon Consumer Products Corporation, New York, NY 10017 (US)
(72) Inventor: SCANCARELLA, Neil. D., Wyckoff, NJ 07481 (US); SANDEWICZ, Robert, W., Monroe Township, NJ 07726 (US); PATIL, Anjali, A., Westfield , NJ 07090 (US); CALELLO, Joseph, F., Bridgewater, NJ 08807 (US)
(74) Representative: Horner, Martin Grenville
(86) International application number: PCT/US2002/005669
(87) International publication number: WO 2002/067877

(56) References cited:
- EP-A- 0 887 073
- US-A- 6 045 782
- US-A- 6 074 654
- US-A- 6 153 206

## Description

### 1. Technical Field

The invention is in the field of lipstick compositions, methods for application, and related articles.

### 2. Background of the Invention

One of the common problems with lipstick is that it must be frequently reapplied as the color wears off very quickly, particularly when eating or drinking. A number of years ago, transfer resistant lipsticks were introduced. Such lipsticks were both long wearing in that they exhibited substantially improved adherence to the lips and at the same time did not transfer off when the lips came into contact with objects such as eating utensils or cups. While transfer resistant lipsticks filled the need gap for longer wearing lipsticks, they created another need gap which was the desire for lip compositions that were more moisturizing to the lips. In particular, many consumers, particularly older women, perceived that transfer resistant lip formulas were more drying on the lips than their traditional lipsticks. While they liked the long wearing and transfer resistant benefits, they were not completely happy with the perceived lack of hydration. In general moisturizing lipsticks generally require the presence of emollient oils, which are precisely the ingredients that compromise long wear and transfer resistance. In other words, in order to optimize wear and provide transfer resistance, the oil level in the lipstick must be closely regulated because oils are marvelous solvents for the resins typically used to achieve these benefits. Solvating the resins, in turn, means that wear and transfer resistance is compromised. Thus, cosmetic companies are still trying to achieve optimal wear, transfer resistance, and hydration in one lip product.

One approach to this problem is set forth in U.S. Patent Nos. 6,074,654; 6,071,503; and 6,019,962 assigned to Procter & Gamble. These patents describe a two pack system where the transfer resistant lipcolor is first applied to the lips and allowed to dry. Then, a second emollient layer in the form of a fatty oil based stick, is applied over the transfer resistant film. Patentees claim that this waxy overcoat composition improves the performance of the transfer resistant layer. However, the wear results for such a product appear to vary in consistency between individual so that wear and other aesthetics are somewhat unpredictable.

Another type of lipstick overcoat, disclosed in U.S. Patent No. 5,750,094, is an example of an overcoat for a standard non-transfer resistant lipstick composition. This overcoat is designed to be compatible with the underlying color coat in that the silica and similar particles present combine with the oil found in the color coat and together, both compositions react to form a more transfer resistant lip coating. The difficulty with this approach is that the overcoat is both reactive and in some sense compatible with the color layer, and if used with a transfer resistant lipstick would interact with it and impact both wear and transfer resistance.

US Patent No. 6,045,782 discloses a transfer resistant anhydrous cosmetic stick composition and a method for providing same. There is no disclosure of coating the disclosed composition to improve the aesthetics thereof.

US 6,153,206 describes the need for a cosmetic stick composition which provides a shiny finish on the lips and at the same time does not compromise feel, stick integrity and transfer resistance. There is no disclosure however of a method for improving the aesthetics of said lipstick composition by coating said composition.

EP-A-0 887 073 describes a cosmetic composition containing as ingredient, at least one fluoroalkylsilicone, however there is no disclosure of the composition containing crosslinked resinous silicone nor a method for improving the aesthetics of the described composition by coating said composition.

Accordingly, it has been discovered that achieving transfer resistance and improved aesthetics in a two pack system, requires that the transfer resistant and overcoat compositions exhibit a very specific and unique relationship. In particular, the transfer resistant color coating must adhere to the lips and provide long wear and resist transfer. The overcoat must be capable of application to the film, and while it should be non-reactive with the film, at the same time it should be capable of interaction with the film to a degree sufficient to adequately wet the film. It is only when the film is adequately wetted that the lipstick composition provides optimal feel, hydration, and comfort.

### 3. Summary of the Invention

It is an object of the invention to provide a method for improving aesthetics such as feel, comfort, gloss, hydration, water resistance, etc., of transfer resistant films on the lips.

It is also an object of the invention to provide a method for wetting a pigmented, transfer resistant film on the lips, which in turn provides a cosmetic composition with improved aesthetics.

It is another object of the invention to provide a multipack cosmetic composition for application to the lips contained in at least two separate receptacles in a single stock keeping unit, said first receptacle containing a pigmented transfer resistant composition, and said second receptacle containing a non-reactive wetting agent composition that serves to wet said transfer resistant composition. The non-reactive wetting agent composition may be in the form of a liquid composition that is applied to the lips, or alternatively, it may be formulated into a semi-solid paste or solid stick composition.

It is another object of the invention to provide for a transfer resistant or long wearing cosmetic composition that provides good feel, comfort, water resistance, and lip hydration to the wearer.

It is another object of the invention to provide a multipack lip treatment system which provides color, hydration, and conditioning to the lips.

According to a first aspect of the present invention there is provided a method for improving the aesthetics of a pigmented, transfer resistant film on the lips, said film comprising at least one crosslinked resinous silicone, a volatile solvent comprising isododecane, and pigment, said method comprising coating said transfer resistant film with a liquid polymeric hydrocarbon having a number average molecular weight of greater than 650 that is nonreactive with but interactive to the transfer resistant film.

The present invention further provides a multipack cosmetic composition comprising at least two separate receptacles in a single stock keeping unit, said first receptacle containing a pigmented transfer resistant composition comprising at least one crosslinked resinous silicone, a volatile solvent comprising isododecane and pigment, and said second receptacle containing a nonreactive liquid wetting agent for said transfer resistant composition, which wetting agent is a liquid polymeric hydrocarbon having a number average molecular weight of at least 650 that is nonreactive with but interactive to the transfer resistant composition.

Thus, the invention comprises a method for improving the aesthetics of a pigmented transfer resistant film on the lips comprising coating the transfer resistant film with a non-reactive wetting agent composition which may be in the form of a liquid where the wetting agent is the only ingredient present or the wetting agent is one of a mixture of ingredients present, or a solid or semi-solid composition where the liquid wetting agent comprises an ingredient, or mixture of wetting agent ingredients, in the composition. The wetting agent composition serves to wet the transfer resistant film, which in turn provides improved hydration, comfort, and wear to the composition on the lips.

### 4. Brief Description of the Drawings

**Figure 1:** depicts a cross section of a lip surface having a transfer resistant lip composition and wetting agent applied thereto, and illustrates how the wetting agent composition used in the method of the invention wets the transfer resistant film by providing a smooth, even coating on the internal and external surfaces of the transfer resistant film.
**Figure 2:** depicts a cross section of a lip surface having a transfer resistant lip composition and wetting agent composition applied thereto, and illustrates a situation where the wetting agent composition does not wet the transfer resistant film, evidenced by beads on the internal and external surfaces of the film.
**Figure 3:** is an illustration of one type of multipack cosmetic composition in accordance with the invention wherein the transfer resistant lip composition and the wetting agent composition are maintained in separate containers but in a single stock keeping unit.
**Figure 4:** is an illustration of another type of multipack cosmetic composition in accordance with the invention wherein the transfer resistant lip composition and the wetting agent composition are kept in separate receptacles within a single container.
**Figure 5:** illustrates another embodiment of the invention where the multipack cosmetic composition contains three separate receptacles in a single stock keeping unit for housing the transfer resistant lip composition, the wetting agent, and a remover composition.
**Figure 6:** illustrates another embodiment of the invention where the multipack cosmetic composition contains three separate containers in a single stock keeping unit for housing the transfer resistant lip composition, the wetting agent composition, and a remover composition.

### 5. Detailed Description

### 5.1 THE METHODS OF THE INVENTION

The invention comprises a method for both wetting, and thereby improving the aesthetics of a pigmented, transfer resistant film on the lips, comprising coating said transfer resistant film with a non-reactive wetting agent composition that serves to wet the transfer resistant film. The non-reactive wetting agent composition may be a liquid wetting agent alone, or a liquid wetting agent composition that contains, in addition to the liquid wetting agent, other ingredients, and the final composition is a liquid, semi-solid, or solid at room temperature (25 °C.). In addition the wetting agent composition may contain a liquid wetting agent in combination with other ingredients that provide a final composition that is in a semi-solid or solid form (such as a paste or stick).

### 5.1.1. Definitions.

The term "improving the aesthetics" means improving qualities such as feel on the lips, hydration, long-wear, gloss, transfer resistance, comfort of the color coat on the lips, water resistance, and general commercial attributes of the composition.

The term "wetting agent composition" means a composition that contains a liquid wetting agent alone, or in combination with other ingredients. The term "liquid" means that the wetting agent is a liquid at room temperature. The wetting agent composition (which contains the liquid wetting agent) may be in the form of a liquid, semi-solid, or solid.

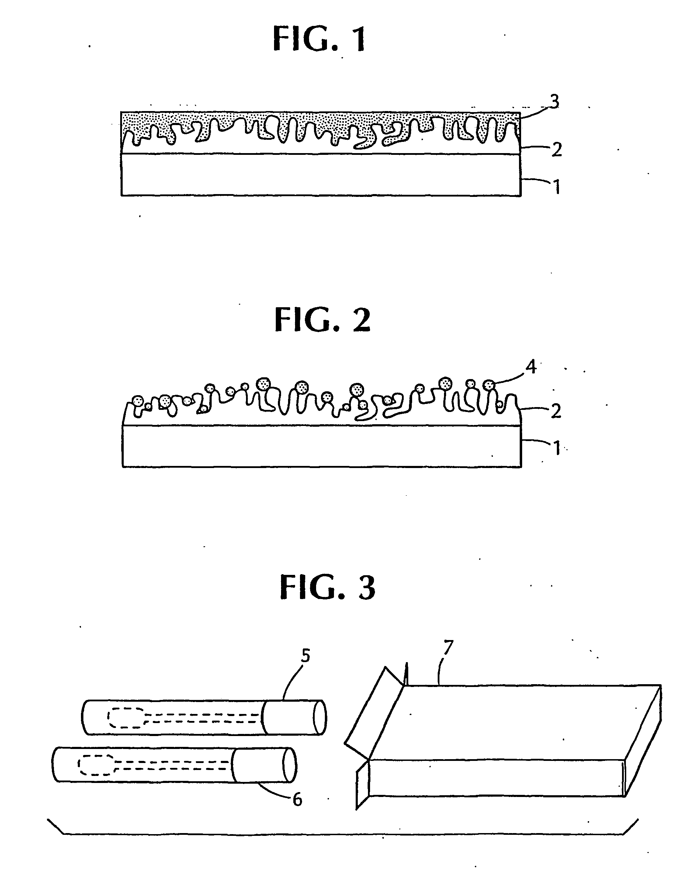

The term "wetting" means the ability of the transfer resistant film to be wet by the wetting agent composition. In accordance with this invention, the wetting agent composition is capable of adequately wetting the transfer resistant film. If the wetting agent composition is a fluid, the fluid flows smoothly over the internal and external film surfaces and any matrices that may exist within the film, and forms a smooth surface with no beading. In other words, if the wetting agent composition beads on the surfaces of the transfer resistant film, or within the matrices, it is not a suitable wetting agent for use in the claimed method. If the wetting agent composition is in the form of a semi-solid or solid composition, the liquid wetting agent present in the composition also serves to wet the transfer resistant film in the same manner, e.g. as the solid ingredients in the composition are softened by contact with the lips, the liquid wetting agent wets plasticizes the transfer resistant film. This is illustrated in Figures 1 and 2. Figure 1 depicts a cross section of the lip surface 1, a transfer resistant film 2 applied thereon, and the wetting agent composition 3 which is applied to the film. The wetting agent composition has properly wetted the film, as is evident in the manner in which the wetting agent has evenly covered the film surfaces. Figure 2 illustrates where the wetting agent composition does not adequately wet the transfer resistant film. The wetting agent composition has formed beads 4 on the transfer resistant film 2, and the film surfaces are not evenly covered.

In general wetting is more scientifically described by a measurement referred to as a "wetting angle" which describes the angle that exists between a specific liquid and a specific solid surface, and which gives an indication of the relative values of the forces of adhesion and cohesion that result in interfacial tension. In general, small wetting angles mean large wettability and large wetting angles mean small wettability. More specifically, if the wetting angle between a specific solid and a specific liquid is small, the liquid wets the solid to a more appreciable degree, and the adhesive forces of the liquid for the solid surface are greater than the internal cohesive forces of the liquid. Conversely, when the wetting angle between a specific solid and a specific liquid is relatively large, the liquid does not wet the solid to an appreciable degree, and the internal cohesive forces of the liquid are greater than the adhesive forces between the liquid and that particular solid surface. It is in the latter instance where beading is often seen. Preferably, in the method of the invention, when the wetting agent composition is applied to the transfer resistant film, the wetting angle between the wetting agent composition and the transfer resistant film surface is less than about 45 degrees.

The term "transfer resistant film" means a film that, when applied to lips, exhibits minimal transfer from the lips when the kiss test, as described in U.S. Patent No. 5,505,937, is performed. In particular, transfer resistance is assessed by applying the film to the lips and allowing it to dry for 5 minutes. Then, the lips are lightly touched to the back of the hand. If no color or a trace of color remains on the back of the hand, the film is considered transfer resistant.

The term "non-reactive" means that the wetting agent composition does not react with any of the ingredients in the transfer resistant film, by, for example, solvating or solubilizing any ingredient in the film, or otherwise reacting at any chemical level with any ingredient in the transfer resistant film.

The term "interactive" means that while the wetting agent composition is non-reactive with the transfer resistant film, it is still capable of physically interacting with the internal and external surfaces of the film by seeping into any matrices, and coating the surfaces of the transfer resistant film, and thereby wetting the film but at the same time being non-reactive and with the transfer resistant film.

### 5.2. The Lip Composition Used to Prepare the Transfer Resistant Film

The transfer resistant film that forms the color coating on the lips in the method of the invention is formed when a transfer resistant pigmented lip composition is applied to the lips. This composition generally comprises a volatile solvent in combination with pigments. The composition may additionally contain a polymeric film former.

### 5.2.1. Polymeric Film Formers

Generally, the lip composition comprises one or more polymeric film formers that are capable of forming a transfer resistant film on the lips when formulated into a pigment containing lip composition. Such compositions typically contain from about 0.1-50%, preferably 0-5-40%, more preferably 1-35% by weight of the total composition of polymeric film formers. Suitable polymeric film formers include synthetic polymers such as silicones and polymers made from ethylenically unsaturated monomers. Also suitable are natural polymers such as gums, resins, and similar materials derived from natural sources.

Suitable silicones include linear and cross-linked silicones that are resinous in character, e.g. which exhibit properties generally associated with resins such as film forming capability, substantivity, and occlusiveness. Examples of cross linked silicones suitable for use are siloxy silicate polymers having the following general formula:

[(RR'R")₃SiO_{1/2}]ₓ [SiO₂]_{y}

wherein R, R' and R" are each independently a C 1-10 straight or branched chain alkyl or phenyl, and x and y are such that the ratio of (RR'R")₃SiO_{1/2} units to SiO₂ units is 0.5 to 1 to 1.5 to 1. Preferably R, R' and R" are a C1 -6 alkyl, and more preferably are methyl and x and y are such that the ratio of (CH₃)₃SiO_{1/2} units to SiO₂ units is 0.75 to 1. Most

[(RR'R")₃SiO_{1/2}]ₓ [SiO₂]_{y}

wherein R, R' and R" are each independently a C1-10 straight or branched chain alkyl or phenyl, and x and y are such that the ratio of (RR'R")₃SiO_{1/2} units to SiO₂ units is 0.5 to 1 to 1.5 to 1. Preferably R, R' and R" are a C1-6 alkyl, and more preferably are methyl and x and y are such that the ratio of (CH₃)₃SiO_{1/2} units to SiO₂ units is 0.75 to 1. Most preferred is this trimethylsiloxy silicate containing 2.4 to 2.9 weight percent hydroxyl groups which is formed by the reaction of the sodium salt of silicic acid, chlorotrimethylsilane, and isopropyl alcohol. The manufacture of trimethylsiloxy silicate is set forth in U.S. patent nos. 2,676,182; 3,541,205; and 3,836,437. Trimethylsiloxysilicate as described is available from GE Silicones under the tradename SR-1000, which is a solid particulate material. Also suitable is Dow Corning 749 which is a mixture of volatile cyclic silicone and trimethylsiloxysilicate.

Also suitable are linear, high molecular weight silicones that are semi-solids or solids at room temperature. Examples of such silicones include dimethicones having viscosities ranging from 500,000 to 10 million, dimethicone copolyols, or silicone waxes such as C14-30 alkyl dimethicone waxes such as ceryl or stearyl dimethicone, or behenoxydimethicone. Preferably, the compositions are free of silicone gums.

Also suitable are silicone esters as disclosed in U.S. Patent Nos. 4,725,658 and U.S. patent no. 5,334,737. Such silicone esters comprise units of the general formula RₐR^{E}_{b}SiO_{[4-(a+b)2]} or R¹³ₓR^{E}_{y}SiO_{1/2}, wherein R and R¹³ are each independently an organic radical such as alkyl, cycloalkyl, or aryl, or, for example, methyl, ethyl, propyl, hexyl, octyl, decyl, aryl, cyclohexyl, and the like, a is a number ranging from 0 to 3, b is a number ranging from 0 to 3, a+b is a number ranging from 1 to 3, x is a number from 0 to 3, y is a number from 0 to 3 and the sum of x+y is 3, and wherein R^{E} is a carboxylic ester containing radical. Preferred R^{E} radicals are those wherein the ester group is formed of one or more fatty acid moieities (e.g. of about 2, often about 3 to 10 carbon atoms) and one or more aliphatic alcohol moieities (e.g. of about 10 to 30 carbon atoms). Examples of such acid moieties include those derived from branched-chain fatty acids such as isostearic, or straight chain fatty acids such as behenic. Examples of suitable alcohol moieties include those derived from monohydric or polyhydric alcohols, e.g. normal alkanols such as n-propanol and branched-chain etheralkanols such as (3,3,3-trimethylolpropoxy)propane. Preferably the ester subgroup (i.e. the carbonyloxy radical) will be linked to the silicon atom by a divalent aliphatic chain that is at least 2 or 3 carbon atoms in length, e.g. an alkylene group or a divalent alkyl ether group. Most preferably that chain will be part of the alcohol moiety, not the acid moiety. Such silicones may be liquids or solids at room temperature.

Other synthetic polymeric film formers that may be used for the lip composition are polymers made from various types of ethylenically unsaturated monomers, including esters of acrylic or methacrylic acid, or ethylene homo- or copolymers, or styrene homo- or copolymers. Such polymers may be homopolymer, copolymers, or graft or block copolymers. Examples of such polymeric film formers are disclosed in U.S. 6,143,283 and 6,153,206. U.S. Patent No. 6,143,283 teaches acrylic polymers that are adhesives at room temperature. One type of such polymer comprises a backbone of vinyl, methacrylic, or acrylic monomers and pendant siloxane and fluorochemical groups, for example Poly(isobutyl methacrylate)-co- methyl FOSEA sold by 3M Company under the tradename SA 70-5 IBMMF. Also suitable are vinyl silicone graft or block copolymers, such as poly(dimethylsiloxane)-g-poly(isobutyl) methacrylate sold by 3M Company under the tradename VS 70. Also suitable are the methacrylate copolymers set forth in U.S. Patent No. 6,153,206, which comprise uncrosslinked synthetic polymers comprising a first repeat unit selected from methacrylate ester monomers which, if polymerized, would yield a polymer having a glass transition temperature of -10 to 75°C., and a second repeat unit selected from methacrylate ester monomers which, if polymerized, would yield a polymer having a glass transition temperature of 76 to 120°C. Examples of such copolymers include those where the first repeat unit is selected from isobutyl methacrylate, ethyl methacrylate, hexyl methacrylate, and mixtures thereof; and the second repeat unit is selected from methyl methacrylate, isobornyl methacrylate or mixtures thereof.

Various types of natural film forming polymers may be used as well. Examples of such polymers include cellulosics and derivatives thereof, gums such as gum arabic, and resins obtained from trees and plants.

Preferably, the transfer resistant film in the method of the invention is formed with a lip composition containing a synthetic polymeric film former that is a silicone resin, in particular, trimethylsiloxysilicate.

### 5.2.2. Solvents

Typically the lip composition contains one or more ingredients that act as solvents for the polymeric film former. The polymeric film former generally remains dissolved in the solvents while the lip composition is stored in the container, but when it is applied to the lips the solvent tends to dissipate, at least in part, permitting the polymeric film former to form a transfer resistant film on the surface of the lips. Typical ranges of solvent are from about 1-98%, preferably 2-95°/a, more preferably 15-85% by weight of the total composition. Suitable solvents are volatile and are liquids at room temperature. The term "volatile" means that the oil has a measurable vapor pressure, or a vapor pressure of at least 2 mm. of mercury at 20 °C. Suitable volatile solvents generally have a viscosity of 0.5 to 10 mm²/second (0.5 to 10 centistokes) at 25°C. Suitable volatile oils include linear silicones, cyclic silicones, paraffinic hydrocarbons, or mixtures thereof.

Cyclic silicones are of the general formula: 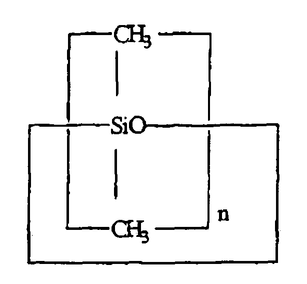 where n = 3-7.

Linear volatile silicones in accordance with the invention have the general formula:

(CH₃)₃Si-O-[Si(CH₃)₂-O]ₙ-Si(CH₃)₃

where n=0-6, preferably 0-5.

Linear and cyclic volatile silicones are available from various commercial sources including Dow Corning Corporation and General Electric. The Dow Corning volatile silicones are sold under the tradenames Dow Corning 244, 245, 344, and 200 fluids. These fluids comprise octamethylcyclotetrasiloxane, decamethylcyclopentasiloxane, hexamethyldisiloxane, and mixtures thereof

Also suitable as the volatile oils are various straight or branched chain paraffinic hydrocarbons having 5 to 40 carbon atoms, more preferably 8-20 carbon atoms. Suitable hydrocarbons include pentane, hexane, heptane, decane, dodecane, tetradecane, tridecane, and C8-20 isoparaffins as disclosed in U.S. patent nos. 3,439,088 and 3,818,105. Preferred volatile paraffinic hydrocarbons have a molecular weight of 70-225, preferably I 60 to 190 and a boiling point range of 30 to 320, preferably 60-260 °C., and a viscosity of less than 10 mm²/second (10 cs.) at 25°C. Such paraffinic hydrocarbons are available from EXXON under the ISOPARS trademark, and from the Permethyl Corporation. Suitable C12 isoparaffins are manufactured by Permethyl Corporation under the tradename Permethyl 99A. Another C12 isoparaffin (isododecane) is distributed by Presperse under the tradename Permethyl 99A. Various C16 isoparaffins commercially available, such as isohexadecane (having the tradename Permethyl R), are also suitable. A particularly preferred solvent for use in the transfer resistant lipstick composition is isododecane.

### 5.2.3. Pigments

The lip composition used to form the transfer resistant film in the method of the invention also comprises pigments. The term "pigment" when used herein means both pigments and powder materials traditionally used for muting color or providing bulk. Typically such transfer resistant compositions contain 0.5-50%, preferably 7-45%, more preferably 10-40%, by weight of the total composition, of pigments having a particle size of 0.02 to 100, preferably 0.5 to 100, microns. Examples of pigments include bismuth oxychloride, titanated mica, fumed silica, spherical silica, polymethylmethacrylate, polyethylene, polypropylene, micronized teflon, boron nitride, acrylate copolymers, aluminum silicate, aluminum starch octenylsuccinate, bentonite, calcium silicate, cellulose, chalk, corn starch, diatomaceous earth, fuller's earth, glyceryl starch, hectorite, hydrated silica, kaolin, magnesium aluminum silicate, magnesium trisilicate, maltodextrin, microcrystalline cellulose, rice starch, silica, talc, mica, titanium dioxide, zinc laurate, zinc myristate, zinc rosinate, alumina, attapulgite, calcium carbonate, calcium silicate, dextran, kaolin, nylon, silica silylate, silk powder, sericite, soy flour, tin oxide, titanium hydroxide, trimagnesium phosphate, walnut shell powder, or mixtures thereof. The above mentioned powders may be surface treated with lecithin, amino acids, mineral oil, silicone oil or various other agents either alone or in combination, which coat the powder surface and render the particles more lipophilic in nature.

Various organic and inorganic pigments that may be used include azo, indigoid, triphenylmethane, anthraquinone, and xanthine dyes which are designated as D&C and FD&C blues, browns, greens, oranges, reds, yellows, etc. Organic pigments generally consist of insoluble metallic salts of certified color additives, referred to as the Lakes, in particular the Lakes of D&C and FD&C colors. Inorganic pigments include iron oxides, ultramarines, chromium, chromium hydroxide colors, and mixtures thereof.

### 5.2.4. Thickening or Gelling Agents

The lip compositions used to form the transfer resistant film may also comprise one or more thickening or gelling agents which impart increased viscosity to the final composition. Typically the compositions comprise 0.1-40%, more preferably 1-15%, more preferably 1-10% by weight of the total composition of such agents. Examples of thickening agents include natural or synthetic waxes, natural or synthetic metal silicates, and derivatives thereof. Examples of synthetic silicate gelling agents are disclosed in U.S. Patent No. U.S. Patent No. 3,586,478. Natural silicates are typically extracted from quartz or dolomite and are often referred to as "clay". Particularly preferred are natural silicates such as montmorillonite minerals and quaternized derviatives thereof. The term "quaternized derivatives" means that the montmorillonite minerals are reacted with one or more quaternary ammonium compounds such as quaternium-18, quaterniwn-16, etc. Examples of suitable waxes include those set forth in U.S. Patent No.5,725,845. Particularly preferred are quaternized montmorillonite minerals including quaternium-18 hectorite. or quaternium-18 bentonite.

### 5.2.5. Other Ingredients

The lip compositions used to form the transfer resistant film may also contain other ingredients such as preservatives, antioxidants, extracts, and so on, so long as these ingredients do not impair the transfer resistant characteristics of the composition.

The lip compositions may be in the form of liquids, solids, or semi-solids. Preferably, the lip composition that forms the transfer resistant film is in the liquid or semi-solid form.

### 5.3. Application of the Transfer Resistant Film

The lip composition as described above is applied to the lips in the usual manner. The manner of application will depend on whether the lip composition is in the form of a solid, liquid, or semi-solid. If in the solid form, the lip composition is probably in the form of a stick, or solidified into a pan with an accompanying applicator. If in the semi-solid or liquid form, the lip composition is probably is dispensed from a vial or similar container using a doe foot applicator or brush. After the lip composition is applied to the lips it is allowed to dry for the appropriate period of time. Drying time will depend on the type of polymeric film former and other ingredients in the composition. Generally, a drying time of ½ minute to 10 minutes is suitable to permit the transfer resistant layer to adequately set on the lips. While the best results are obtained if the lip composition is allowed to dry completely prior to application of the wetting agent composition, it is not necessary. In most cases, the transfer resistant layer that is formed on the lips does not feel like normal lipstick. The film may feel tight and dry depending on the polymeric film former used in the transfer resistant composition.

### 5.4. The Wetting Agent Composition

The wetting agent composition that is applied to the transfer resistant film in the method of the invention is a liquid wetting agent ingredient or combination of ingredients including the wetting agent that are non-reactive with the transfer resistant film but at the same time capable of interacting with the internal and external surfaces and matrices of the film by seeping into spaces and surfaces in a smooth, even fashion, to a degree sufficient to wet the transfer resistant film. A variety of wetting agents may be used in the composition so long as they fulfill the criteria described herein.

### Hydrocarbons

Suitable are polymeric hydrocarbons preferably having number average molecular weights greater than about 650. Examples of such polymers include polybutenes such as Indopol H-100, Indopol H-50, and Parapol 700, manufactured by Amoco. Also suitable are polymeric alpha olefins obtained by the polymerization of alpha olefins. Particularly preferred are Synton PAO 100 manufacutured by Uniroyal Chemical, Puresyn 150 and Puresyn 100, manufactured by ExxonMobil Chemical. Puresyn 150, 100, and 300 are referred to as hydrogenated polydecene.

The wetting agent may consist of only one ingredient or a mixture of ingredients in the form of a composition, provided the wetting agent compound or composition contains the specifications mentioned herein and remains non-reactive, yet interactive with the transfer resistant film.

Preferably, the wetting agent has a refractive index ranging from 1.0 to 1.5

### 5.5. The Wetting Agent Composition

If desired the liquid wetting agent may be incorporated into a liquid, semi-solid, or solid composition wherein additional ingredients may be present, such as waxes, oils, humectants, colorants, preservatives, antioxidants, and so on. Suitable amounts of wax range from about 0.01-40% by weight of the total composition. Such waxes may be animal, vegetable, mineral, or synthetic waxes, and will generally have a melting point ranging from about 30 to 150°C. Preferred waxes include synthetic wax, polyethylene, microcrystalline waxes, mineral waxes, silicone waxes, and so on, as disclosed in U.S. Patent No. 5,505,937. Suitable ranges of oils (other than the liquid wetting agent) are about 0.1-50% by weight of the total composition and include volatile and non-volatile silicone oils, esters, triglycerides, and so on. Suitable ranges of humectants are from about 0.1-20% by weight of the total composition and include glycerin, polyols, glyceryl esters, and so on.

### 5.6. Application of the Wetting Agent

The transfer resistant lip composition is first applied to the lips. Preferably, this film is permitted to dry for at least 30 seconds to 5 minutes although this is not completely necessary. In fact, in some cases, application of the wetting agent to the transfer resistant film without permitting the transfer resistant film to dry first will provide wetting that is equivalent to that achieved when the film is permitted to dry. The wetting agent composition does not interfere with the transfer resistant character of the lip composition because it is selected so as to be non-reactive and with the transfer resistant film. However, the wetting agent composition will cause improved wetting of the film, which in turn improves the aesthetics. The improved aesthetics include hydration, comfort, feel, gloss. The wetting agent composition will wear away during the day or when eating or drinking, and may simply be reapplied at desired intervals in order to maintain the transfer resistant film in a comfortable state. The transfer resistant film will remain on the lips for an extended period of time, possibly even until removed with a chemical remover. If the transfer resistant film is particularly adherent, certain chemical removers may be used to remove it. Figure 1 depicts how the wetting agent composition should wet the transfer resistant film in accordance with the method of the invention. Figure 2 shows wetting that is inadequate. In particular, the wetting agent composition has formed beads on the film surface and within the matrices.

### 6. THE MULTIPACK COSMETIC COMPOSITION

The invention also comprises a multipack cosmetic composition for application to the lips comprising at least two separate receptacles in a single stock keeping unit, said first receptacle containing a pigmented transfer resistant composition, and said second receptacle containing an non-reactive wetting agent composition for said transfer resistant composition.

The term "multipack cosmetic composition" means a cosmetic composition that is applied in at least two separate steps requiring at least two separate compositions housed in separate containers or separate receptacles.

The term "single stock keeping unit" means that the multipack is sold as a single item with the different cosmetic components in separate receptacles. The receptacles may be in one single container or two or more separate containers sold as a single stock keeping unit. If the latter, preferably, the multipack is scanned under a single UPC code.

The containers may be made of suitable materials generally used for this purpose including plastics such as ABS, styrene, polyethylene and so on. Typically such containers are cylindrical vials with screw cap closures. Generally, the applicators used to apply the products are affixed to the inner top surface of the screw cap closures.

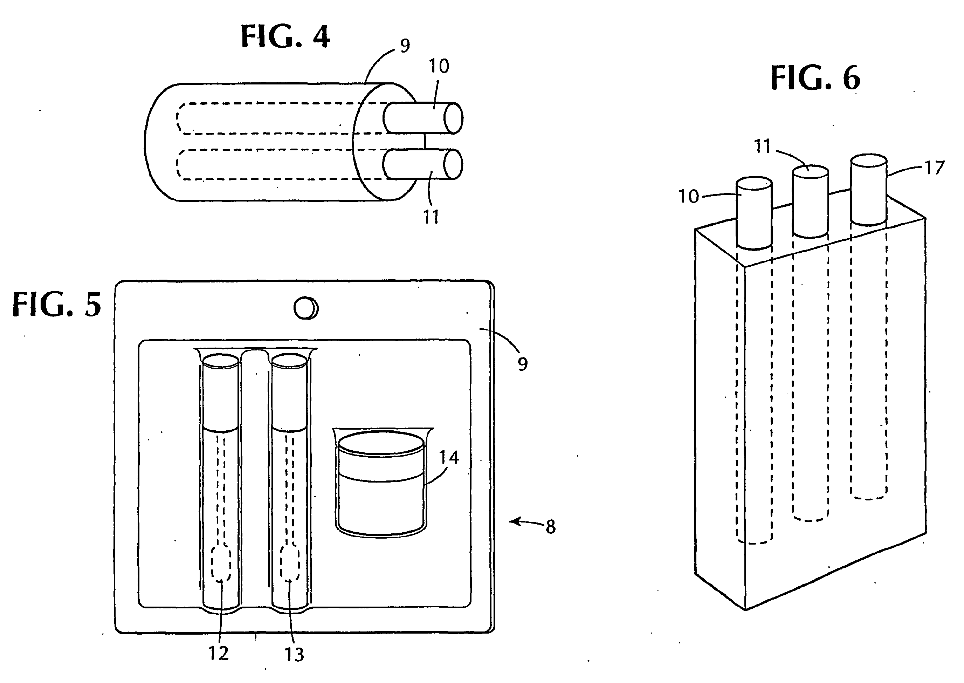

Figure 3 depicts one type of multipack cosmetic composition in accordance with the invention. One receptacle is a separate container 5 which contains the transfer resistant lipstick composition and the other receptacle is a separate container 6 that contains the wetting agent composition. The containers are generally sold together in a single stock keeping unit in the form of a blister pack 8 (see Figure 5) or in a box 7, or other similar unit.

**Figure 4** illustrates another multipack cosmetic composition in accordance with the invention. The multipack cosmetic composition is housed in a single container 9 that contains two separate receptacles 10 and 11. One receptacle contains the transfer resistant lip product and the other receptacle contains the wetting agent composition.

Figure 5 illustrates another embodiment of the invention showing a multipack cosmetic composition comprised of three containers for housing the transfer resistant lip product 12, the wetting agent 13, and a remover composition 15. The remover composition 15 is used to remove the lipstick from the lips. The three containers may be sold in a box (see Figure 3) or in a blister pack 8 where the three containers are affixed to the blister card 9 by means of vacuum formed plastic 10 in the usual fashion. '

Figure 6 illustrates yet another embodiment of the invention where the transfer resistant lipstick composition 14, the wetting agent composition 15, and the remover composition 16 are housed in a single container having three separate receptacles.

The multipack cosmetic composition of the invention enables the consumer to obtain a long wearing, transfer resistant lip finish which exhibits all of the aesthetics mentioned. This attributes are achieved because the film is adequately wetted by the wetting agent composition, and at the same time the wetting agent composition is non-reactive and with the transfer resistant film so it does not solvate or otherwise chemically react with the film. The lip color film will remain on the lips for extended periods of time, and will generally not transfer to eating utensils, glassware, and the like upon contact. The wetting agent composition will eventually wear off the lips, but is reapplied at desired intervals to maintain the aesthetic benefits. The wetting agent composition provides a protective film over the transfer resistant film in addition to the other benefits mentioned.

The invention will be further described in connection with the following examples, which are set forth for the purposes of illustration only.

### EXAMPLE 1

A transfer resistant color coat was prepared according to the following formula:

| | w/w% |
|---|---|
| Trimethylsiloxysilicate/isododecane (50/50) | 31.5 |
| Quaternimn-18 hectorite/isododecane | |
| /propylene carbonate (87/10/3) | 20.00 |
| Isododecane | 27.50 |
| FD&C Red #7 in isododecane (28%) | 10.00 |
| Mica | 7.00 |
| Synthetic wax | 2.50 |
| Fluorosilicone | 1.50 |

All ingredients except the fluorosilicone were combined and heated to a temperature of 85 to 90 °C. and mixed well. The mixture was cooled to room temperature. The fluorosilicone was added and the mixture heated again to a temperature of 85 to 90° C. until the fluorosilicone had become well mixed. The mixture was cooled to room temperature. The composition, a semi-solid paste, was put into a pot.

### EXAMPLE 2

The color coat of Example 2 was tested with the following overcoats. The color coat was applied to the lips and allowed to dry. The overcoat was applied and allowed to set for 15 to 30 minutes. The overcoat was then wiped with a tissue. The amount of color that transferred to the tissue was evaluated on a 0 to 3 scale, with the - sign in front of the number indicating that the overcoat performed slightly worse than the number following it.

### Rating

0 No transfer (wetting agent non-reactive and interactive with transfer resistant film)
1 Some color transfer (wetting agent reactive and compatible with transfer resistant film)
2 Substantial amount of color transfer (wetting agent reactive and compatible with transfer resistant film)
3 Basecoat removed (wetting agent reactive and compatible with transfer resistant film)

### OVERCOAT RATING

| *OILS* (comparative example) | |
|---|---|
| Ricinus Communis seed oil | 1 |
| Lanolin oil | -1 |
| Apricot kernel oil | 3 |
| Sesame oil | 3 |
| Sweet almond oil | 3 |
| Synthetic jojoba oil | 3 |
| Tricaprylin this is an ester liquid | 3 |

| *HYDROCARBONS* | |
|---|---|
| Polybutene (Indopol H-100 Mn* 940) | 1 |
| Polybutene (Indopol H-15 Mn 600) | 3** |
| Polybutene (Indopol H-50 Mn 815) | 1 |
| Polybutene (Indopol L-14 Mn 370 and L-50 Mn 455) | 3 ** |
| Polybutene (Parapol 450 Mn 420) | 3 ** |
| Polybutene (Parapol 700 Mn 700) | -1 |
| Polyalphaolefin (Synton PAO 100) | 1 |
| Hydrogenated polyisobutene | 3 |
| Decene/butene copolymer | 3 |

| *POLYURETHANES*** | |
|---|---|
| PPG 51/SMDI copolymer (Polyprepolymer 14) | 2 |
| PEG-2 soyamine/IPDI copolymer (Polyderm PPI-SA) | 3 |
| PEG 15 cocamine/IPDI copolymer (Polyderm PPI-CO-15) | 2 |
| Glycereth-7-diglycerol PEG-15 cocamine/IPDI copolymer (Polyderm PPI-G7/CA) | 2 |
| Polydiethylene glycol adipate/IPDI copolymer (Polyderm PPI-PE) | 2 |
| Castor oiL/IPDI copolymer (Polyderm PPI-CO) | 2 |

| *SURFACTANTS*** | |
|---|---|
| PEG-25 hydrogenated castor oil | 2 |
| PPG-24 glycereth-24 | 3 |
| PEG-40 sorbitan peroleate | 3 |
| PEG-7 glyceryl cocoate | 3 |
| Polysorbate 80 | 2 |
| PPG-stearyl alcohol | 3 |
| PPG-10 methyl glucose ether | 1 |

| *ESTEAS*** | |
|---|---|
| Triacetin | -1 |
| Acetyl tributyl citrate | 3 |
| PPG-2 dibenzoate | 2 |
| Isononyl isononanaoate | 3 |
| Trioctyl dodecyl citrate | 3 |
| Isostearyl hydroxy stearate | 3 |
| Diisostearyl maleate | 3 |
| Pentaerythritol tetraoctanoate | 3 |
| Triisostearyl citrate | 2 |
| Diisostearyl adipate | 3 |
| Diisopropyl sebacate | 3 |

| *MISCELLANEOUS*** | |
|---|---|
| Phytantriol | 3 |
| C10-30 cholesterol/lanosterol esters | 2 |
| Abietic alcohol | 2 |
| Octyl dodecanol | 3 |
| Propylene carbonate | 2 |
| PVPlhexadecene copolymer | 2 |
| Dimethicone fluid (350 cs) | 3 |
| Dimethicone copolyol (Dow Corning 1248 fluid) | 3 |
| Dimethicone gum in cyclomethicone (Dow Corning 1401 fluid) | 3 |
| Fluorosilicone (trifluoroethyl C1-4 dimethicone) | 3 |
| Glycerine | 1 |
| Propylene glycol | 1 |
| Butylene glycol | 1 |
| Polyvinylpyrrolidone | 1 |
| PVP/VA copolymer | 1 |
| Glyceryl polymethacrylate | 1 |
| PVP/carbamyl polyglycol ester | 1 |
| Polyperfluoropolymethylisopropyl ether (Fomblin HC/R) | 1 |
| | |

| | |
|---|---|
| ** Number average molecular weight.* ** are comparative examples | |

### EXAMPLE 3

Transfer resistant lipcolor formulas were made as follows:

| | *w*/*w%* | |
|---|---|---|
| | 1 | 2 |
| Polymer solution1 | 92.0 | 45.00 |
| PPG-2 dibenzoate | 3.75 | -- |
| D&C Red #7 in lanolin oil2 | 4.25 | 10.00 |
| Dimethicone (0.65 cs.) | - | 30.00 |
| VS-703 | -- | 15.00 |

1 A solution of 50% by weight of a copolymer comprised of isobutyl methacrylate and isobornyl methacrylate and 50% by weight of isododecane.
2 33% by weight D&C Red #7 and 67% by weight lanolin oil,
3 A solution of 45% by weight poly(dimethylsiloxane)-g-poly(isobutyl methacrylate) and 55% isododecane, 3M Company.

The color coats 1 and 2, above, were tested with the following overcoats. The color coat was applied to the lips and allowed to dry. The overcoat was applied and allowed to set for 15 to 30 minutes. The overcoat was then wiped with a tissue. The amount of color that transferred to the tissue was evaluated on the same 0 to 3 scale as set forth in Example 2.

| OVERCOAT | RATING - Formula 1 | RATING - Formula 2 |
|---|---|---|
| Polyperfluoropolymethylisopropyl ether (Fomblin HC/R)** | 0 | 0 |
| Ricinus Communis seed oil** | 3 | 3 |
| Polyalphaolefin (Synton PAO 100) | -0 | 0 |
| Diisostearyl malate ** | 0 | 1 |
| Sweet almond oil** | 3 | 3 |
| PPG-10 methyl glucose ether** | 0 | 0 |
| Polybutene (Indopol L-14) | | |
| Mn 370** | 2 | 3 |
| Polybutene (Indopol H50) | | |
| Mn 815 | 1 | 0 |
| Dimethicone 350 cs | -0 | 0 |
| Fluorosilicone** | 0 | 0 |
| Dimethicone copolyol (Dow Corning 1248 Fluid)** | 0 | 0 |
| Dimethicone gum in cyclomethicone (Dow Corning 1401 Fluid) ** | -2 | 0 |

| | | |
|---|---|---|
| ** comparative examples ** comparative examples | | |

### EXAMPLE 4

A wetting agent composition in the solid form in accordance with the invention was made as follows:

| | *w*/*w%* |
|---|---|
| Polyethylene | 10.25 |
| Cyclomethicone | 25.50 |
| Polyalphaolefin (Puresyn 150)* | 39.85 |
| Polyalphaolefin (Puresyn 100)** | 24.00 |
| Triclosan | 0.10 |
| Benzoic acid | 0.20 |
| Butylated hydroxy toluene | 0.1 |

| | |
|---|---|
| **hydrogenated polydecene, M W. 3,500* ***hydrogenated polydecene MW 2,900* | |

The composition was prepared by combining the ingredients with sufficient wanning, mixing well, and pouring into stick molds and allowing to cool.

## Claims

1. A method for improving the aesthetics of a pigmented, transfer resistant film on the lips, said film comprising at least one crosslinked resinous silicone, a volatile solvent comprising isododecane, and pigment, said method comprising coating said transfer resistant film with a liquid polymeric hydrocarbon having a number average molecular weight of greater than 650 that is nonreactive with but interactive to the transfer resistant film.

2. The method of claim 1, wherein the aesthetics improved are feel, comfort, transfer resistance, hydration, long wear, water resistance and gloss.

3. The method of claim 1 or claim 2, wherein the crosslinked resinous silicone is trimethylsiloxy silicate.

4. The method of any preceding claim, wherein the film further comprises one or more nonvolatile silicones, esters or fatty alcohols.

5. The method of any preceding claim, wherein the polymeric hydrocarbon is an alpha olefin.

6. The method of any preceding claim wherein the polymeric hydrocarbon is polybutene or polydecene.

7. The method according to claim 1, wherein said method is for wetting said pigmented, transfer resistant film on the lips.

8. The method of claim 7, wherein the crosslinked resinous silicone is trimethylsiloxy silicate.

9. The method of claim 7 or claim 8, wherein the polymeric hydrocarbon is an alpha olefin.

10. The method of any one of claims 7 to 9, wherein the polymeric hydrocarbon is polybutene or polydecene.

11. A multipack cosmetic composition comprising at least two separate receptacles in a single stock keeping unit, said first receptacle containing a pigmented transfer resistant composition comprising at least one crosslinked resinous silicone, a volatile solvent comprising isododecane and pigment, and said second receptacle containing a nonreactive liquid wetting agent for said transfer resistant composition, which wetting agent is a liquid polymeric hydrocarbon having a number average molecular weight of at least 650 that is nonreactive with but interactive to the transfer resistant composition.

## Patentansprüche

1. Verfahren zum Verbessern der Ästhetik eines pigmentierten, transferresistenten Films auf den Lippen, wobei besagter Film umfasst: wenigstens ein vernetztes Silikonharz, ein flüchtiges Lösungsmittel, welches Isododecan umfasst, und Pigment, wobei besagtes Verfahren das Beschichten des besagten transferresistenten Films mit einem flüssigen polymeren Kohlenwasserstoff umfasst, der ein Zahlengewichtsmittel des Molekulargewichts von größer als 650 aufweist, und der gegenüber dem transferresistenten Film nichtreaktiv sondern interaktiv ist.

2. Verfahren nach Anspruch 1, wobei die verbesserte Ästhetik Gefühl, Annehmlichkeit, Transferresistenz, Hydration, lange Haltbarkeit, Wasserbeständigkeit und Glanz sind.

3. Verfahren nach Anspruch 1 oder 2, wobei das vernetzte Silikonharz Trimethylsiloxisilikat ist.

4. Verfahren nach einem der vorstehenden Ansprüche, wobei der Film weiter ein oder mehrere nichtflüchtige Silikone, Ester oder Fettalkohole umfasst.

5. Verfahren nach einem der vorstehenden Ansprüche, wobei der polymere Kohlenwasserstoff ein α-Olefin ist.

6. Verfahren nach einem der vorstehenden Ansprüche, wobei der polymere Kohlenwasserstoff Polybuten oder Polydecen ist.

7. Verfahren nach Anspruch 1, wobei besagtes Verfahren zum Benetzen des besagten pigmentierten, transferresistenten Films auf den Lippen dient.

8. Verfahren nach Anspruch 7, wobei das vernetzte Silikonharz Trimethylsiloxisilikat ist.

9. Verfahren nach Anspruch 7 oder 8, wobei der polymere Kohlenwasserstoff ein α-Olefin ist.

10. Verfahren nach einem der Ansprüche 7 bis 9, wobei der polymere Kohlenwasserstoff Polybuten oder Polydecen ist.

11. Kosmetische Zusammensetzung als Multipackung, welche mindestens zwei getrennte Aufnahmebehälter in einer einzigen Einheit zum Aufbewahren von Material umfasst, wobei besagter erster Aufnahmebehälter eine pigmentierte transferresistente Zusammensetzung enthält, welche umfasst: mindestens ein vernetztes Silikonharz, ein flüchtiges Lösungsmittel, welches Isododecan umfasst, und Pigment, und besagter zweite Aufnahmebehälter ein nichtreaktives flüssiges Benetzungsmittel für besagte transferresistente Zusammensetzung enthält, wobei das Benetzungsmittel ein flüssiger polymerer Kolilenwasserstoff ist, der ein Zahlengewichtsmittel des Molekulargewichts von mindestens 650 aufweist, und der gegenüber der transferresistenten Zusammensetzung nichtreaktiv sondern interaktiv ist.

## Revendications

1. Procédé permettant d'améliorer l'esthétique d'un film résistant au transfert, pigmenté sur les lèvres, ledit film comprenant au moins une silicone résineuse réticulée, un solvant volatil comprenant de l'isododécane, et un pigment, ledit procédé comprenant le revêtement dudit film résistant au transfert avec un hydrocarbure polymérique liquide, ayant une masse moléculaire moyenne supérieure à 650, qui ne réagit pas, mais qui est interactif avec le film résistant au transfert.

2. Procédé selon la revendication 1, dans lequel l'esthétique améliorée inclut le toucher, le confort, la résistance au transfert, l'hydratation, la longue tenue, la résistance à l'eau et le brillant.

3. Procédé selon la revendication 1 ou 2, dans lequel la silicone résineuse réticulée est le silicate de triméthylsiloxy.

4. Procédé selon l'une quelconque des revendications précédentes, dans lequel le film comprend en outre au moins une silicone, un ester ou de l'alcool gras non volatils.

5. Procédé selon l'une quelconque des revendications précédentes, dans lequel l'hydrocarbure polymérique est une alpha-oléfine.

6. Procédé selon, l'une quelconque des revendications précédentes, dans lequel l'hydrocarbure polymérique est le polybutène ou le polydécène.

7. Procédé selon la revendication 1, dans lequel ledit procédé permet d'humidifier ledit film résistant au transfert, pigmenté sur les lèvres.

8. Procédé selon la revendication 7, dans lequel la silicone résineuse réticulée est le silicate de triméthylsiloxy.

9. Procédé selon la revendication 7 ou 8, dans lequel l'hydrocarbure polymérique est une alpha-oléfine.

10. Procédé selon l'une quelconque des revendications 7 à 9, dans lequel l'hydrocarbure polymérique est le polybutène ou le polydécène.

11. Composition cosmétique à emballage multiple comprenant au moins deux réceptacles séparés dans une seule unité de conservation du contenu, ledit premier réceptacle contenant une composition résistante au transfert, pigmentée, comprenant au moins une silicone résineuse réticulée, un solvant volatil comprenant l'isododécane et le pigment, et ledit deuxième réceptacle contenant un agent mouillant liquide non réactif pour ladite composition résistante au transfert, lequel agent mouillant est un hydrocarbure polymérique liquide ayant une masse moléculaire moyenne d'au moins 650, qui ne réagit pas, mais qui est interactif avec la composition résistante au transfert.
